# EUROPÄISCHE PATENTSCHRIFT

(11) **EP 1 338 528 B1**
(45) Veröffentlichungstag und Bekanntmachung des Hinweises auf die Patenterteilung: **12.03.2008**
(21) Anmeldenummer: 03002940.9
(22) Anmeldetag: 26.02.1999
(51) Int. Cl.: B65D 75/58, B65D 83/14, B65D 83/00

(54) **Verwendung eines Behälters für eine medizinische Flüssigkeit**
Use of a container for medical liquid
Utilisation d'un récipient pour liquide médical

(30) Priorität: 27.02.1998 DE 19808295
(43) Veröffentlichungstag der Anmeldung: 27.08.2003
(62) Teilanmeldung aus: 99910294.0
(73) Patentinhaber: Boehringer Ingelheim International GmbH, 55216 Ingelheim am Rhein (DE)
(72) Erfinder: Freund, Bernhard, 55435 Gau-Algesheim (DE); Essing, Martin, 44141 Dortmund (DE); Kladders, Heinrich, 54568 Muehlheim (DE); Zierenberg, Bernd, 55411 Bingen am Rhein (DE); Geser, Johannes, 55218 Ingelheim (DE); Reinecke, Holger, 44229 Dortmund (DE); Eicher, Joachim, 44227 Dortmund (DE); Hochrainer, Dieter, 57392 Oberkirchen (DE)

(56) Entgegenhaltungen:
- EP-A- 0 585 908
- EP-A- 0 622 311
- WO-A-95/15895
- WO-A-96/03218
- WO-A-97/12687
- WO-A-97/26998
- PATENT ABSTRACTS OF JAPAN vol. 1998, no. 01, 30. Januar 1998 (1998-01-30) & JP 09 225356 A (YOSHINO KOGYOSHO CO LTD), 2. September 1997 (1997-09-02)

## Beschreibung

Die Erfindung betrifft die Verwendung eines Behälters für eine medizinische Flüssigkeit, der gas- und flüssigkeitsdicht ist.

In EP - 0 182 094 A2 ist eine flaschenförmige Verpackung angegeben, die aus einem formsteifen Außenbehälter und einem darin befindlichen Innenbehälter besteht, der als leicht verformbarer Beutel für das Füllgut ausgebildet ist. Der Vorformling wird durch Koextrusion zweier koaxialer schlauchförmiger Abschnitte hergestellt. Die beiden Abschnitte bestehen aus zwei thermoplastischen Kunststoffen, die keine Verbindung miteinander eingehen. Der Vorformling wird in einer Blasform aufgeweitet. Am flachen Boden des Innenbehälters ist eine Schweißnaht vorhanden. Der am Außenbehälter angeformte flache Boden enthält eine schlitzförmige Öffnung. Im Bereich der Entnahmeöffnung sind Außenbehälter und Innenbehälter formschlüssig miteinander verbunden. Diese Verpackung wird im wesentlichen in einem Arbeitsgang hergestellt.

Das Füllgut wird mittels einer in der Entnahmeöffnung angebrachten Pumpe entnommen, wobei sich der Innenbehälter unter Abnahme seines Volumens verformt. Durch den offenen Schlitz im flachen Boden des Außenbehälters tritt Luft in den Raum zwischen Außenbehälter und verformtem Innenbehälter ein, wodurch die Entstehung eines Unterdruckes in diesem Zwischenraum verhindert wird. Der Innenbehälter hat - außer im Bereich der Entnahmeöffnung - keinen festen Kontakt zum Außenbehälter. Die Verpackung kann mit einem fast bis zum flachen Boden reichenden Tauchrohr versehen sein, das den Innenbehälter in gestreckter Lage hält. Diese Verpackung ist nur bei bestimmter räumlicher Lage einwandfrei benutzbar und vollständig entleerbar.

In EP - 0 620 165 A1 wird ein Schlauchbeutel aus Verbundfolie beschrieben. Die Verbundfolie besteht zumindest aus einer außenliegenden Kunststoff-Folie und einer innenliegenden Metallfolie. Der Schlauchbeutel ist an beiden Enden sackartig verschlossen. Der Schlauchbeutel ist mit einer Sollbruchstelle versehen, mittels der er sich an dieser Stelle zuverlässig öffnen lässt. Ein derartiger Schlauchbeutel dient zum Lagern einer aushärtbaren Masse, die mittels eines Auspressgerätes aus dem Schlauchbeutel ausgepresst wird.

EP - 0 068 653 A1 beschreibt einen zur einmaligen Benutzung vorgesehenen aus einer Folie hergestellten flexiblen und kollabierbaren Behälter, der in einer mehrfach verwendbaren Saugflasche eingesetzt wird. Das eine Ende des Behälters ist offen, das andere Ende ist mittels einer Schweißnaht verschlossen und mit einer Lasche versehen. Die Lasche ist in einem am Boden der Saugflasche vorhandenen Spalt eingeklemmt. Dadurch wird der beutelartige Behälter in der Saugflasche ständig in gestreckter Lage gehalten.

EP 0 654 419 beschreibt einen Behälter zur Aufbewahrung von flüssigen Arzneimitteln, wobei die Düse zur Aufnahme des Arzneimittels durch den Patienten von der Ruheposition in eine zweite Position zur Abgabe des Arzneimittels gebracht wird.

Aufgabe der vorliegenden Erfindung ist, die Verwendung eines Behälters für eine medizinische Flüssigkeit anzugeben, der gas- und flüssigkeitsdicht ist, dessen Füllvolumen an den vorgesehenen Verwendungszweck anpasst ist, der bei geringem Unterdruck in vorgegebener Weise plastisch und irreversibel kollabiert und annähernd vollständig entleerbar ist.

Gegenstand der Erfindung ist die Verwendung eines gas- und flüssigkeitsdichten Behälters für eine medizinische Flüssigkeit in einem treibgasfreien Zerstäuber, welcher einen Entnahmestutzen in Form eines Hohlkolbens aufweist, wobei der Behälter einen Folienbeutel, der an beiden Enden verschlossen ist,umfaßt wobei mindestens ein Ende durch eine Schweißnaht verschlossen ist, die im wesentlichen quer zur Beutelachse verläuft und der Folienbeutel bei einem Differenzdruck zwischen dem Innenraum des Behälters und seiner Umgebung unterhalb 300 hPa (300 mbar) durch den Außendruck verformbar ist,
- einen formstabilen Flansch, der an dem Folienbeutel dicht angebracht ist und der auf einen Entnahmestutzen aufgesteckt wird,
- einen Führungskanal in dem Flansch,
- wobei in dem Führungskanal eine Dichtstelle ausgebildet ist und/oder eine Presspassung, die den Entnahmestutzen umschließt,
- und eine Entnahmestelle für die Flüssigkeit im Bereich des Führungskanals, in die bei Gebrauch der Hohlkolben sticht, so daß er in die medizinische Flüssigkeit eintaucht.

In einer weiteren Ausführungsform der erfindungsgemäßen Verwendung des Behälters kann der kollabierbare Folienbeutel bereits bei einem Differenzdruck unterhalb 150 hPa (150 mbar) oder bevorzugt unterhalb 80 hPa (80 mbar) durch den Außendruck verformt werden und kollabieren.

Weiterhin kann bei der erfindungsgemäßen Verwendung der Behälter die im folgenden beschriebenen Merkmale aufweisen:

Der Folienbeutel kann an beiden Enden durch eine Schweißnaht verschlossen sein. In diesem Fall ist der formstabile Flansch an der Seite des Folienbeutels, bevorzugt in der Nähe eines Endes des Folienbeutels, dicht angeschweißt. Der Folienbeutel kann jedoch auch an einem Ende durch eine Schweißnaht und am anderen Ende durch den formstabilen Flansch dicht verschlossen sein. In diesem Fall ist das eine Ende des Folienbeutels mit dem formstabilen Flansch, bevorzugt auf dessen Umfang, verschweißt.

Der formstabile Flansch kann verschieden geformt sein. Falls er am Ende des Folienbeutels als dessen Verschluss angebracht ist, kann er rotationssymmnetrisch geformt und an die Größe des Endes des Folienbeutels angepasst sein. Der formstabile Flansch kann mit einem Führungskanal versehen sein, in den der Entnahmestutzen eingeführt wird und in dem sich der Entnahmestutzen bei aufgestecktem Behälter befindet.

Es kann zweckmäßig sein, den Führungskanal mit einer Preß passung zu versehen, die den Entnahmestutzen umschließt. Die Preßpassung kann ein Abschnitt des Führungskanals sein, der aus einer glatten Innenwand besteht mit einem Innendurchmesser, der von dem Außendurchmesser des Entnahmestutzens nur wenig abweicht. In einer weiteren Ausführungsform können in einem Abschnitt des Führungskanals auf dessen Innenwand mehrere Ausbuchtungen vorhanden sein. Die Ausbuchtungen können zum Beispiel drei in axialer Richtung verlaufende symmetrisch angeordnete und länglich geformte Ausbuchtungen sein. Weiter können mehrere, in einem axialen Abstand voneinander angeordnete und in azimutaler Richtung verlaufende Ausbuchtungen vorgesehen sein, die zum Beispiel zwei Ringe bilden, oder die aus mehreren Ringabschnitten bestehen. Ferner können die Ausbuchtungen wendelförmig verlaufen; sie können aus mehreren auf der Innenwand des Führungskanals verteilten Wendelabschnitten oder aus einem Wendelabschnitt bestehen, dessen Länge größer ist als der Umfang des Führungskanals. Eine derartige Preßpassung ermöglicht das Aufstecken des Behälters auf den Entnahmestutzen sowie einen hinreichend festen Sitz des formstabilen Flansches auf dem Entnahmestutzen. Ferner kann der Behälter nach seiner Entleerung von dem Entnahmestutzen abgezogen werden, ohne den Entnahmestutzen zu beschädigen.

Der formstabile Flansch besteht aus Gummi, Metall oder Kunststoff, bevorzugt aus einem thermoplastischen Kunststoff. Es kann zweckmäßig sein, den formstabilen Flansch aus demselben Kunststoff herzustellen, aus dem der Folienbeutel oder die Innenseite des Folienbeutels besteht.

Die Schweißnaht an einem oder beiden Enden des Folienbeutels kann U-, V- oder T-förmig ausgebildet sein; sie verläuft im wesentlichen quer zur Beutelachse. Sie kann sich teilweise in Richtung der Beutelachse erstrecken, wodurch beim Entnehmen von Flüssigkeit die definierte Verformung des Folienbeutels begünstigt wird.

Innerhalb oder an einem der Enden des Führungskanals kann eine Dichtstelle vorgesehen sein. Die Dichtstelle kann aus einem Ring bestehen, der in einer auf der Innenwand des Führungskanals angebrachten Nut liegt. Der Querschnitt des Ringes kann O-förmig oder im Wesentlichen rechteckig sein. Der Ring ist gegebenenfalls mit einer Dichtlippe versehen. Der Ring kann aus einem Elastomer, aus einem thermoplastischen Elastomer oder aus Gummi bestehen. Die Dichtstelle verschließt den Füllraum des auf den Entnahmestutzen aufgesteckten Behälters gegen die Umgebungsluft gas- und flüssigkeitsdicht. Sie erlaubt das Abziehen des entleerten Behälters von dem Entnahmestutzen. Die Dichtstelle ist erforderlich, falls die Dichtwirkung der Preßpassung nicht ausreicht.

Die Entnahme ist bevorzugt als Einstichstelle ausge bildet. An der Einstichstelle kann eine durchstechbare Membran vorgesehen sein, die beim Aufstecken des Behälters auf den Entnahmestutzen durchstochen wird. Die Membran ist bevorzugt zwischen der Dichtstelle und dem Flüssigkeitsraum im Folienbeutel angeordnet. Die durchstechbare Membran kann an einem der Enden oder innerhalb des Führungskanals angebracht sein. Sie ist bevorzugt unmittelbar an dem Ende des Führungskanals oder in der Nähe dieses Endes angebracht, das dem Flüssigkeitsraum zugewandt ist. Sie kann ein Teil des formstabilen Flansches oder ein Teil des Folienbeutels sein. Falls sie ein Teil des formstabilen Flansches ist, kann sie gleichzeitig mit dem formstabilen Flansch hergestellt werden. Sie kann aus demselben Kunststoff wie der formstabile Flansch bestehen. Die durchstechbare Membran wirkt als originaler Verschluss für den Füllraum des Folienbeutels.

In einer weiteren Ausführungsform kann die Entnahmestelle mit einer Siegelfolie versiegelt sein, die vor dem Aufstecken des Behälters auf den Entnahmestutzen abgezogen wird, oder die beim Aufstecken des Behälters auf den Entnahmestutzen durchstochen wird.

Der formstabile Flansch kann einteilig oder mehrteilig sein. Der mehrteilige Flansch kann bevorzugt zweiteilig sein. Das außenliegende Teil des Flansches ist mit dem Folienbeutel dicht verbunden. Das äußere Teil enthält eine Öffnung, die mit dem inneren Teil dicht verschlossen ist. Beide Teile können mittels eines Gewindes miteinander verschraubt werden, oder mittels einer Schnappverbindung oder durch Ultraschallschweißen miteinander verbunden werden. Der einteilige Flansch ist analog zum zweiteiligen Flansch ausgebildet, er enthält jedoch keine Verbindungselemente.

Der formstabile Flansch kann gleichzeitig mit Preßpassung, Nut für die Dichtstelle und durchstechbarer Membran hergestellt werden.

Der Folienbeutel kann aus einem Schlauch bestehen, der keine in axialer Richtung des Folienbeutels verlaufende Schweißnaht aufweist. Ferner kann er aus einer Folie hergestellt werden und eine oder zwei in Längsrichtung verlaufende Schweißnähte haben. Er kann als Flachbeutel oder als Beutel mit Seitenfalten ausgebildet sein. Ein Beutel mit einer, in Längsrichtung verlaufenden Schweißnaht wird bevorzugt.

Die Schweißnähte am Folienbeutel können von 0,7 mm bis 3 mm breit sein; ihre Breite wird entsprechend den an Dichtheit und Haltbarkeit der Naht gestellten Forderungen gewählt. Breite Längsnähte am Folienbeutel können nach dem verschweißen umgebogen werden, damit sie außen am Folienbeutel etwa anliegen und der Folienbeutel nur wenig breiter ist als seine Breite im unverschweißten Teil zwischen den Schweißnähten.

Der Folienbeutel kann aus einer Folie aus Metall oder Metall-Legierung - bevorzugt aus Aluminium, Gold oder Kupfer - oder aus Kunststoff - bevorzugt einem Thermoplasten - bestehen. In einer anderen Ausführungsform kann der Folienbeutel aus einer Verbundfolie aus Kunststoff und Metall bestehen. Die Verbundfolie besteht bevorzugt aus zwei oder drei miteinander verbundenen Folien. Wei ter kann der Folienbeutel aus einer Kunststoff-Folie bestehen, auf die eine Schicht aus Metall, Glas oder Keramik, zum Beispiel durch Aufdampfen, aufgebracht ist. Die Folien aus Kunststoff oder aus Metall sind einige Mikrometer dick. Die Dicke der Aufdampf schichten aus Metall, Glas oder Keramik liegt im Submikrometerbereich.

Die Verbundfolie aus zwei Folien kann aus einer Metallfolie und einer Kunststoff-Folie bestehen, die miteinander verbunden sind. Die Metallfolie bildet die Innenseite oder die Außenseite der Verbundfolie. In einer anderen Ausführungsform besteht die Verbundfolie aus zwei unterschiedlichen Kunststoffen.

Die Verbundfolie aus drei Folien besteht bevorzugt aus zwei Kunststoff-Folien, zwischen denen eine Folie aus Metall liegt. Alle drei Folien sind miteinander verbunden. An Stelle der Metallfolie kann eine Schicht aus Glas oder Keramik vorhanden sein, zum Beispiel aus Siliziumoxid (SiOₓ), die auf eine Kunststoff-Folie aufgedampft ist.

In einer weiteren Ausführungsform besteht die Innenfolie der Verbundfolie aus einem Copolymer, zum Beispiel einem Polyethylen-Copolymer aus Ethylen-Acrylsäure. Für die äußere Kunststoff-Folie der Verbundfolie wird bevorzugt ein Kunststoff verwendet, zum Beispiel Polyethylen-terephthalat, dessen Schmelztemperatur größer ist als die Schmelztemperatur des Kunststoffs der Innenfolie. Hierdurch wird beim Herstellen des Folienbeutels das nahtweise Verschweißen des Kunststoffs der Innenfolie erleichtert.

Bei der Verbundfolie kann zwischen zwei Folien gegebenenfalls eine Haftvermittlerschicht vorhanden sein.

Der Folienbeutel kann aus einer Kunststoff-Folie mit einer Dicke von 20 µm bis 100 µm bestehen. Weiter kann er aus einer Verbundfolie mit einer Innenfolie aus Kunststoff mit einer Dicke von 20 µm bis 100 µm und einer Außenfolie aus Metall mit einer Dicke von 8 µm bis 20 µm bestehen. Ferner kann er aus einer Verbundfolie mit einer Innenfolie aus Kunststoff mit einer Dicke von 20 µm bis 100 µm, einer Mittelfolie aus Metall mit einer Dicke von 8 µm bis 20 µm und einer Außenfolie aus Kunststoff mit einer Dicke von 10 µm bis 40 µm bestehen.

Die Schweißnähte am Folienbeutel sowie die Schweißstelle zwischen Folienbeutel und formstabilem Flansch werden nach bekannten Verfahren hergestellt, wie thermisches Schweißen, UltraschallSchweißen oder Induktions-Schweißen bei Verbundfolien mit metallener Schicht, wobei die Schweißstellen in erhitzten Zustand bevorzugt zusammengedrückt werden. Derartige Verfahren sind beispielsweise in EP - 0 111 131 und EP - 0 130 239 angegeben.

Ein formstabiler Flansch aus Gummi oder aus Metall kann mit dem Folienbeutel durch Kleben oder gegebenenfalls durch Vulkanisieren verbunden werden.

Der Behälter kann sich in einer formstabilen Hülse aus Metall oder aus Kunststoff befinden, deren eines Ende mit dem formstabilen Flansch lösbar oder unlösbar verbunden ist, und deren anderes Ende gegebenenfalls mit einem Boden verschlossen ist. Die Hülse kann in Wesentlichen rundum geschlossen sein. Sie enthält jedoch mindestens eine Öffnung, oder an der Verbindungsstelle mit dem Flansch befindet sich ein Spalt. Weiter kann die Hülse als formstabiler Korb mit vielen Öffnungen ausgebildet sein. Der Behälter kann sich an Stelle der Hülse in einem formstabilen U-förmigen Bügel befinden, wobei das Ende jedes Schenkels des Bügels an dem formstabilen Flansch befestigt ist und die Schenkel länger sind als der Folienbeutel.

Der in einer Hülse befindliche Behälter ist nur am formstabilen Flansch mit der Hülse verbunden. Das mit einer Schweißnaht verschlossene Ende oder die beiden mit einer Schweißnaht verschlossenen Enden des Folienbeutels sind mit der Hülse nicht verbunden.

Beim Übergang von Flüssigkeit aus dem Behälter in den Entnahmestutzen legt sich der Folienbeutel durch die Wirkung des Außendruckes flach zusammen. Durch die Öffnung in der Hülse oder durch den Spalt zwischen Hülse und formstabilem Flansch tritt Luft in den Raum zwischen der Hülse und dem Folienbeutel ein und führt den Druckausgleich herbei. Damit ist im Folienbeutel kein Ventil erforderlich, und die Flüssigkeit im Folienbeutel kommt nicht mit Luft in Berührung.

Der Folienbeutel ist diffusionsdicht für die medizinische Flüssigkeit und ihre Bestandteile sowie für Gase. Das Material für den Folienbeutel und gegebenenfalls der Aufbau der Verbundfolie werden dementsprechend gewählt. Diffusionsdicht im Sinne der vorliegenden Erfindung bedeutet einen Verlust an Flüssigkeit (gemessen mit Ethanol bei Raumtemperatur) des Behälters durch Diffusion von weniger als 0,6 mg pro Tag, bevorzugt weniger als 0,4 mg pro Tag, besonders bevor weniger als 0,2 mg pro Tag, insbesondere weni ger als 0,1 mg pro Tag.

Die Innenfolie oder die Innenseite des Folienbeutels steht mit der eingefüllten Flüssigkeit in Kontakt. Für diese Folie wird ein Material gewählt, das durch die Flüssigkeit nicht angegriffen wird und durch das die Flüssigkeit nicht beeinträchtigt wird. Diese Folie wird, bevorzugt als schweißfähige Folie ausgelegt.

Eine der Folien oder eine zum Beispiel aufgedampfte Schicht ist die Diffusionssperre, die die Diffusion der Flüssigkeit oder ihrer Bestandteile sowie die Diffusion von Gasen aus dem oder in den Folienbeutel verhindert. Es kann zweckmäßig sein, die Diffusionssperre gegen mechanische Beschädigung und gegen ein Zerreißen der Diffusionssperre beim Biegen der Folie durch eine weitere auf die Diffusionssperre aufgebrachte Kunststoff-Folie zu schützen, damit die Diffusion von Flüssigkeit oder von Gasen dauerhaft verhindert bleibt.

Da der Folienbeutel diffusionsdicht gegen Gase ist, kann der durch die Flüssigkeitsentnahme entstehende Unterdruck im Folienbeutel nicht durch eindiffundierendes Gas ausgeglichen werden, und der Folienbeutel kollabiert zuverlässig auch bei sehr langsamer Entnahme von Flüssigkeit aus dem Behälter. Die Flüssigkeit kann auch in vielen Teilmengen, zum Beispiel 200 Dosierungen, verteilt über eine längere Zeit, zum Beispiel drei Monate, aus dem Folienbeutel entnommen werden.

Der in einer im wesentlichen geschlossenen Hülse befindliche Behälter ist von außen unzugänglich und kann bei der Lagerung und beim Aufstecken auf den Entnahmestutzen nicht beschädigt werden. Die im Wesentlichen geschlossene Hülse oder die als Korb mit vie len Öffnungen versehene Hülse oder der formstabile Bügel erleichtern die Lagerung des Behälters mit dem dünnwandigen Folienbeutel und seine Handhabung beim Aufstecken auf den Entnahmestutzen und beim Abziehen des leeren Behälters von dem Entnahmestutzen.

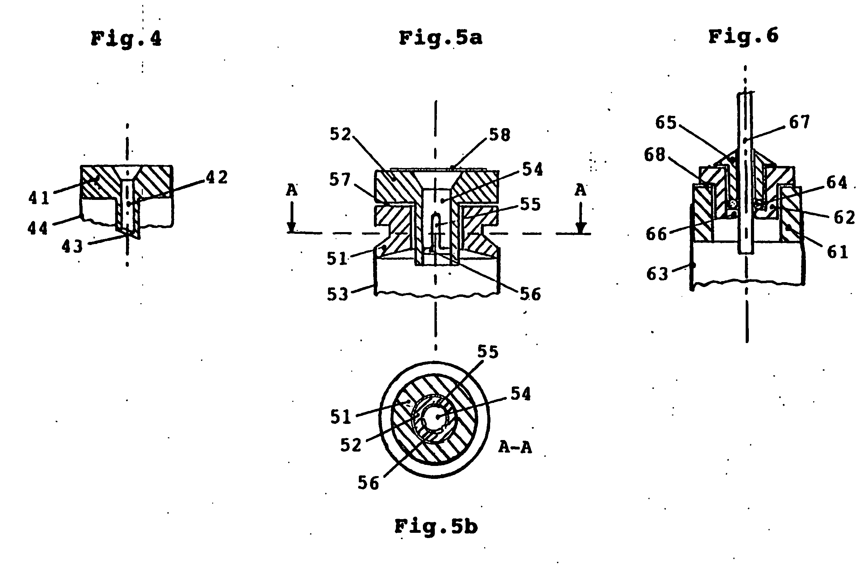

Der Entnahmestutzen ist beispielsweise der Hohlkolben eines Zerstäubers für medizinische Flüssigkeiten. Ein derartiger Zerstäuber ist in DE - 195 36 902.5 und in WO - 97/12687 (speziell in den dortigen Figuren 6a und 6b) beschrieben. Der Hohlkolben dieses Zerstäubers ist als Entnahmestutzen für die in dem Behälter enthaltene medizinische Flüssigkeit ausgebildet. Der Behälter wird auf den bevorzugt in der Achse des Zerstäubers angebrachten Hohlkolben aufgesteckt, wobei das Ende des Hohlkolbens in die Entnahmestelle sticht und damit in die medizinische Flüssigkeit eintaucht. Die Dichtstelle im formstabilen Flansch schließt den Innenraum des Behälters gegen die Außenwand des Hohlkolbens dicht ab. Die Preßpassung kann den Behälter auf dem Hohlkolben mechanisch festhalten.

Es kann zweckmäßig sein, anstelle der oder zusätzlich zur Preßpassung (kraftschlüssigen Verbindung) zwischen Behälter und Entnahmestutzen eine lösbare, formschlüssige Verbindung zwischen dem formstabilen Flansch des Behälters und dem Entnahmegerät, zum Beispiel einem Zerstäuber, vorzusehen. Eine derartige Verbindung kann als steckbare Schnappverbindung aus mehreren Schnapphaken bestehen, die in einem Anschlussstück im Entnahmegerät angebracht sind. Beim Einstecken des Behälters in das Entnahmegerät greifen die Schnapphaken in eine Aussparung im Flansch, zum Beispiel in eine umlaufende Rille oder hinter eine Kante des formstabilen Flansches, ein. Die Schnappnasen sind bevorzugt rund geformt oder in beiden Bewegungsrichtungen des Behälters abgeschrägt, um einen leeren Behälter mit mäßig großem Kraftaufwand entnehmen zu können und einen vollen Behälter in das Entnahmegerät einstecken zu können.

Der Behälter eignet sich besonders als auswechselbare Kartusche für inhalierbare Arzneimittel-Lösungen in treibgasfreien Zerstäubern. Das Füllvolumen des Behälters kann von 0,5 ml bis 5 ml, bevorzugt von 1 ml bis 4 ml und besonders bevorzugt von 1 ml bis 3 ml oder von 2 ml bis 4 ml betragen. Diese Lösungen weiden portionsweise mit einer Dosis jeweils von 10 Mikroliter bis 50 Mikroliter, bevorzugt von 15 µl bis 20 µl entnommen.

Der Hülsendurchmesser kann von 10 mm bis 30mm betragen, bevorzugt von 12 mm bis 17 mm. Die Länge des Behälters einschließlich des aus der Hülse hervorstehenden Teils des formstabilen Flansches kann von 20 mm bis 60 mm, bevorzugt von 30 mm bis 50 mm betragen.

Der Behälter dient als Primärpackmittel zur Aufnahme einer medizinischen Flüssigkeit, zum Beispiel ein in einem Lösemittel gelöstes Arzneimittel. Als Lösemittel sind beispielsweise Wasser, Ethanol oder deren Mischungen geeignet. Als Arzneimittel werden beispielsweise Berotec (Fenoterol-Hydrobromid; 1-(3,5-dihydroxy-phenyl)-2-[[1-(4-hydroxy-benzyl)-ethyl]-amino]-ethanol-hydrobromid), Atrovent (Ipratropiumbromid), Berodual (Kombination aus Fenoterol-Hydrobromid und Ipratropiumbromid),Salbutamol (oder Albuterol),Combivent, Oxivent (Oxitropiumbromid) Ba 679 (Tiotropiumbromid), BEA 2108 (Di-(2-thienyl)-glykolsäuretropenolester), Flunisolid, Budesonid, Beclomethason und andere verwendet.

Die Erfindung betrifft darüber hinaus die Verwendung des Behälters, wobei
- der Zerstäuber ein Entnahmegerät ist, das mit einem Anschlußstück versehen ist,
- in das der Behälter durch eine Schnappverbindung zwischen dem Anschlußstück (154; 161) im Entnahmegerät und dem formstabilen Flansch (148) des Behälters eingesteckt wird.

Die Erfindung betrifft weiterhin die Verwendung des Behälters, wobei
- eine umlaufende Rille (158; 147) im formstabilen Flansch, in die die am Anschlußstück (154; 161) angebrachten Schnappnasen (156; 163) der Schnapphaken (155; 162) bei in das Anschlußstück eingestecktem Behälter eingreifen.

Die Erfindung betrifft weiterhin einen treibgasfreien Zerstäuber welcher einen Entnahmestutzen in Form eines Hohlkolbens (67) zur Entnahme einer Flüssigkeit aufweist, der so mit einem Behälter für eine medizinische Flüssigkeit mit Merkmalen wie oben beschrieben, verbunden ist, dass der Hohlkolben durch die Entnahmestelle hindurch in den Behälter hineinragt.

In WO - 98/27959 werden stabilisierte wässrige Arzneimittel-Zubereitungen zum Herstellen von treibgasfreien Aerosolen für die Inhalation beschrieben. Auf die dort beanspruchten und in den Beispielen angegebenen Formulierungen wird Bezug genommen.

Geeignete Arzneimittel-Zubereitungen in ethanolischer Lösung sind beispielsweise in WO - 97/01329 angegeben, insbesondere wird auf die dort genannten Wirkstoffe (siehe dort die Seiten 2 und 3) sowie die dort beanspruchten stabilisierten Formulierungen Bezug genommen.

Der Behälter hat folgende Vorteile:
- Er ist wirtschaftlich herstellbar, für einmaligen Gebrauch geeignet und benötigt einen nur geringen Materialeinsatz.
- Er ist steril herstellbar, steril befüllbar und steril versiegelbar.
- Er ist für zum Inhalieren vorgesehene Arzneimittel verwendbar, die als Lösungen in Ethanol, Ethanol/Wasser oder Wasser vor liegen.
- Die Flüssigkeit wird steril entnommen; dabei wird keine Luft in den Behälter eingesaugt. Die Flüssigkeit kommt mit Luft, Sauerstoff oder Kohlendioxid nicht in Berührung.
- Der Behälter ermöglicht die gasblasenfreie Entnahme der medizinischen Flüssigkeit.
- Er ist dicht gegen die Diffusion von Flüssigkeiten und Gasen.
- Er ist im befüllten Zustand je nach Arzneimittel über mehrere Jahre lagerfähig und erfüllt die Forderungen sämtlicher amt licher Arzneibücher (Pharmakopöen).
- Er ist bereits bei einem geringen Unterdruck leicht verformbar. Im kollabierten Zustand bleibt er flach und annähernd ge streckt, er behält seine Ausgangslänge nach dem Entleeren annähernd bei.
- Er benötigt kein Ventil für den Druckausgleich nach dem Ent nehmen eines Teils der Flüssigkeit.
- Der Behälter lässt sich weitgehend entleeren, auch bei wechselnder Lage und bei Überkopflage.
- Der Folienbeutel ist nur mit dem formstabilen Flansch ver bunden. Er ist nicht an der gegebenenfalls vorhandenen Hülse befestigt.
- Sein Füllvolumen kann durch Ändern der Länge und/oder des Durchmessers des Folienbeutels leicht auf einen vorgegebenen Wert innerhalb eines gewissen Bereiches eingestellt werden.
- Er kann vor dem Verschließen befüllt werden, bevor die einzige oder die zweite Schweißnaht angebracht wird; ein besonderer Verschluss ist nicht erforderlich.
- Der Behälter kann ohne oder mit einer Hülse verwendet werden.
- Der in einer Hülse befindliche Behälter ist vor äußeren Beschädigungen geschützt.
- Die Flüssigkeit im Folienbeutel ist durch einen undurchsich tigen Folienbeutel oder durch eine rundum geschlossene undurchsichtige Hülse gegen Lichteinwirkung geschützt.
- Er lässt sich auf einfache Weise und ohne Drehbewegung in ein Entnahmegerät einstecken und entnehmen.

Der Behälter wird an Hand der beispielhaften Figuren weiter erläutert.

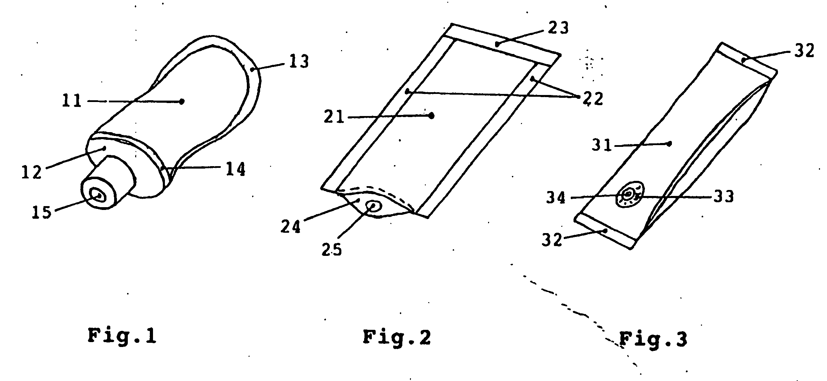

Die Figuren 1 bis 3 zeigen in Schrägansicht verschiedene Ausführungsformen des an beiden Enden verschlossenen Folienbeutels.
Figur 1 zeigt einen Schlauchbeutel (11) mit zylindrischem formstabilen Flansch (12) und U-förmiger Quernaht (13), die das eine Ende des Schlauchbeutels verschließt und sich teilweise in Längsrichtung des Schlauchbeutels erstreckt. Der Rand (14) des Flan sches ist mit dem anderen Ende des Schlauchbeutels verbunden. In der Achse des Flansches befindet sich ein Loch (15), in das ein Entnahmestutzen eingeführt werden kann.
Figur 2 zeigt einen Siegelrandbeutel (21), der aus zwei aufeinanderliegenden Folien besteht. Er hat zwei in Längsrichtung des Beutels verlaufende Schweißnähte (22) und an seinem einen Ende eine in Querrichtung verlaufende Schweißnaht (23). Das andere Ende ist mit einem fischartig geformten formstabilen Flansch (24) verbunden. In der Mitte des Flansches befindet sich ein Loch (25), in das ein Entnahmestutzen eingeführt werden kann.
Figur 3 zeigt einen Seitenfalten-Beutel (31) mit Falten an beiden Längsseiten, der an beiden Enden mit je einer quer verlaufen den Schweißnaht (32) verschlossen ist. Auf einer Flachseite des Beutels ist der formstabile Flansch (33) aufgeschweißt. In das Loch (34) des Flansches kann ein Entnahmestutzen eingeführt wer den.

In den Figuren 4 bis 7 sind verschiedene Ausführungsformen des formstabilen Flansches im Schnitt dargestellt.
Figur 4 zeigt einen Längsschnitt durch einen einstückigen Flansch (41) mit einem als Preßpassung ausgebildeten zylindrischen Führungskanal (42) für einen zylindrischen Entnahmestutzen. Das äußere Ende des Führungskanals ist angeschrägt, das andere Ende ist mit einer zur Flanschachse geneigt angebrachten Membran (43) verschlossen. Dieser Flansch wird in einem Arbeitsgang gefertigt. Der Rand des Flansches ist mit einem Folienbeutel (44) verbunden.
Figur 5 a zeigt einen mehrteiligen zylindrischen Flansch im Längsschnitt und Figur 5b im Querschnitt an der in Figur 5 a mit A-A gekennzeichneten Linie. Das Unterteil (51) ist mit einem Folienbeutel (53) verbunden. Das Oberteil (52) steckt in einer Öffnung des Unterteils. Das Oberteil ist mit einem Führungskanal (54) versehen, in dem drei in Achsenrichtung des Flansches verlaufende längliche Wülste (55) als Preßpassung für einen Entnahmestutzen sowie eine ringförmigen Wulst (56) als Dichtung vorhanden ist. Unterteil (51) und Oberteil (52) sind an ihrer Berührungsfläche (57) miteinander verschweißt. Die Eintrittsöffnung mit Einführungsschräge des Führungskanals ist mit einer Siegelfolie (58) versiegelt.
In Figur 6 ist ein mehrteiliger zylindrischer formstabiler Flansch dargestellt. Das Unterteil (61) ist mit einem Folienbeutel (63) verbunden. Das Oberteil (62) ragt in das ringförmige Unter teil hinein. Im Oberteil befindet sich ein O-Ring (64) als Dicht ung, der durch eine eingepreßte Stopfbuchse (65) gehalten wird. Die Öffnung in der Stopfbuchse dient als Führungskanal für einen Entnahmestutzen. Auf der Innenseite der Dichtung befindet sich am Oberteil eine umlaufende Wulst (66), die als Preßpassung ausgebildet ist. In den Führungskanal ist ein Entnahmestutzen (67) eingeschoben. Der Behälter wird mittels der wulstförmigen Preßpassung (66) auf dem Entnahmestutzen gehalten. Unterteil (61) und Oberteil (62) sind an ihrer Berührungsfläche (68) miteinander verschweißt.

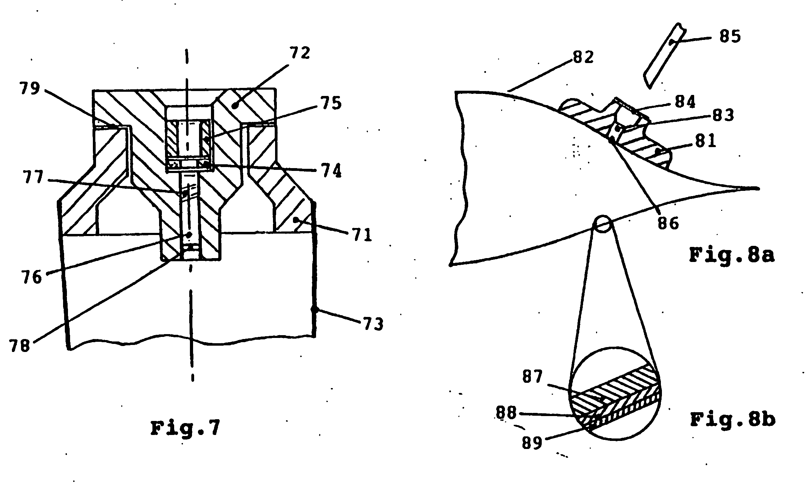

In Figur 7 ist ein weiterer mehrteiliger Flansch dargestellt. Das Unterteil (71) ist mit einem Folienbeutel (73) verbunden. Das Oberteil (72) befindet sich in dem mit einer Schulter versehenen ringförmigen Unterteil. Das Oberteil enthält einen flachen Ring (74) als Dichtung, der durch eine eingepreßte Stopfbuchse (75) gehalten wird. Unterhalb der Flachdichtung befindet sich der Führungskanal (76) für einen Entnahmestutzen. Die Preßpassung besteht aus zwei gewindeartig umlaufenden Wülsten (77) innerhalb des Führungskanals. In der Nähe des unteren Endes des Führungskanals ist eine Membran (78) senkrecht zur Flanschachse angebracht, die beim Aufstecken des Behälters auf den Entnahmestutzen durchstochen wird. Die Membran wird mit dem Oberteil in einem Arbeitsgang herge stellt. Unterteil und Oberteil sind an ihrer Berührungsfläche (79) miteinander verbunden.
In Figur 8 a ist ein einstückiger formstabiler Flansch (81) im Querschnitt dargestellt, der auf der Seite eines Folienbeutels (82) angebracht ist. Der Flansch enthält eine Öffnung (83), die als Preßpassung für einen Entnahmestutzen dient. Dieser Flansch ist auf seiner Außenseite mit einer Siegelfolie (84) versiegelt. Beim Aufstecken des Behälters auf einen Entnahmestutzen (85) mit angeschrägtem Ende wird der Folienbeutel am inneren Ende (86) des Führungskanals durchstochen.
Figur 8b zeigt einen Querschnitt durch eine handelsübliche Laminat-Folie mit drei Schichten, aus der der Folienbeutel be steht. Die Innenfolie (87) besteht aus Polyethylen (40 µm dick), die Mittelfolie (88) ist die Diffusionssperre aus Aluminium (12 µm dick), und die Außenfolie (89) besteht aus Polyethylen-tereph thalat (12 µm dick).

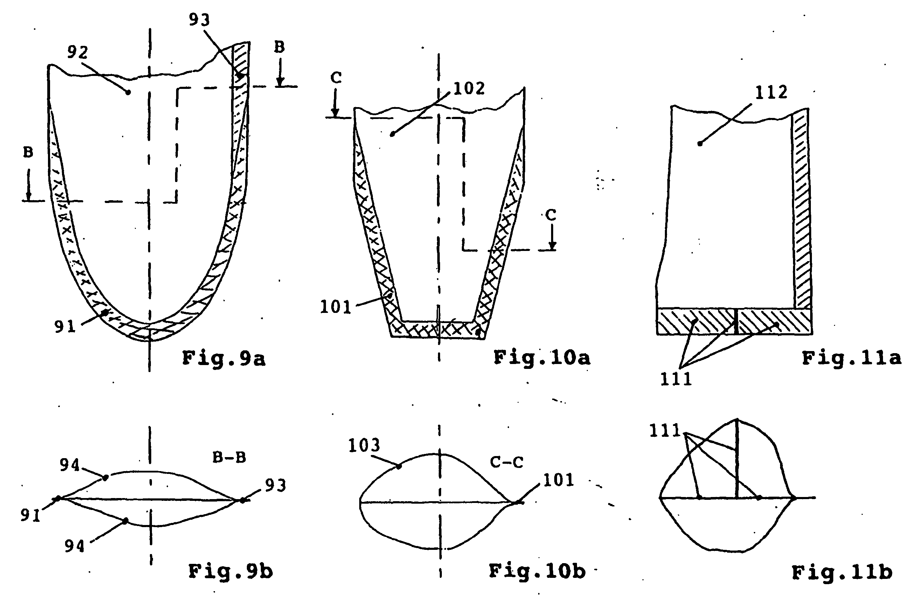

In den Figuren 9 bis 11 sind verschiedene Ausführungen der Schweißnaht, mit der der Folienbeutel an mindestens einem Ende verschlossen ist, in Seitenansicht und im Querschnitt (Figuren 9 b und 10 b) oder in der Aufsicht auf das Beutelende (Figur 11 b) dargestellt.
Figur 9 a zeigt eine U-förmige Schweißnaht (91), die sich teilweise in Längsrichtung des Folienbeutels (92) erstreckt und auf einer Seite in eine in Längsrichtung verlaufende Schweißnaht (93) des Folienbeutels übergeht. Figur 9b zeigt einen Querschnitt durch den Folienbeutel an der in Figur 9a mit B-B gekennzeichneten Linie. In den Schweißnähten ist die Innenschicht der gefalteten Mehrschicht-Folie (94) verschweißt.
Figur 10 a zeigt eine V-förmige Schweißnaht (101) die sich teilweise in Längsrichtung des Folienbeutels erstreckt. In diesem Fall besteht der Folienbeutel aus einer Schlauchfolie ohne Längsnaht. Figur 10b zeigt einen Querschnitt durch den Folienbeutel an der in Figur 10 a mit C-C gekennzeichneten Linie. In der Schweißnaht ist die gefaltete Einschicht-Folie (103) verschweißt.
Figur 11 a zeigt eine T-förmige Schweißnaht (111) in Seitenansicht und Figur 11 b in der Aufsicht auf das verschweißte Ende des Folienbeutels (112). Die drei Schenkel der T-Naht sind insgesamt so lang wie der flach zusammengelegte Folienbeutel außer halb der T-Naht breit ist.

Der mit einer U-, V- oder T-Naht verschweißte Folienbeutel ist im Bereich der Quernaht nicht größer als der Durchmesser der Hülse, in die der Folienbeutel gegebenenfalls eingeschoben wird.

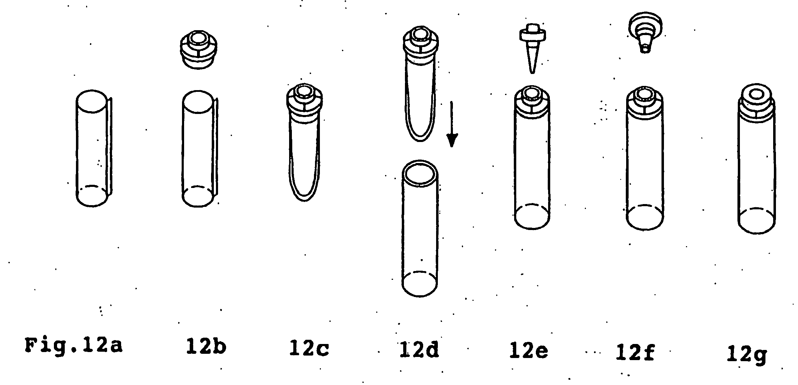

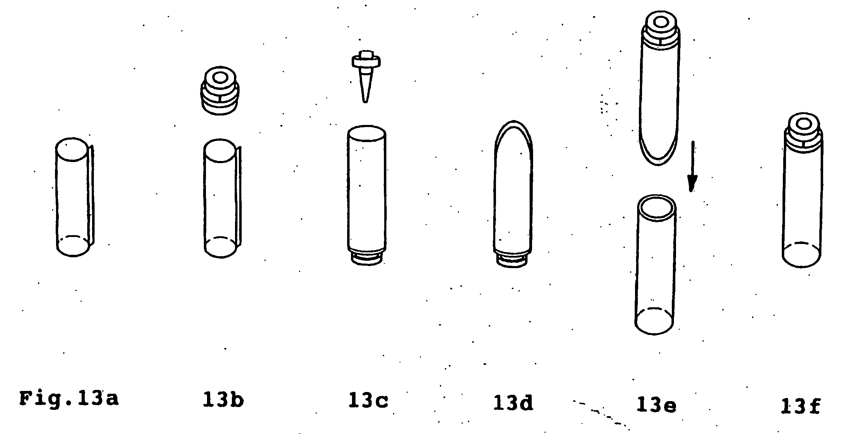

Die Herstellung und Befüllung des Behälters ist in den Figuren 12 und 13 in Schrägansicht schematisch dargestellt.

Ein gefalteter Folienstreifen wird an den geschnittenen Seiten mit einer Schweißnaht in Längsrichtung versehen, in Abschnitte zerteilt und zu einem Schlauch geformt (Figur 12 a). Das im Spritzgußverfahren hergestellte Unterteil eines zweiteiligen zylindrischen Flansches wird mit dem einen Ende eines derartigen Schlauchabschnittes verschweißt. Das andere Ende des Abschnittes wird mit einer U-förmigen Quernaht verschweißt (Figuren 12 b und 12 c). Der fertige Behälter (Figur 12 c) wird in eine zylindrische Hülse aus Aluminium geschoben (Figur 12 d), deren Rand in eine Rille im Rand des zylindrischen Flansches gedrückt wird. Dadurch ist der Behäl ter mit der Hülse fest verbunden. Der in der Hülse befindliche leere Behälter wird durch das mit einer Öffnung versehene Unter teil des Flansches mit einem Fluid befüllt (Figur 12 e). Nach dem Befüllen wird das Oberteil des Flansches in das Unterteil eingedrückt (Figur 12 f), und beide Teile werden miteinander dicht verbunden. Das Fertigteil (Figur 12 g) ist zum Aufstecken auf einen Entnahmestutzen bereit.

Ein weiteres Herstellungsverfahren ist in Figur 13 dargestellt.

Ein Abschnitt eines mit einer Längsnaht versehenen Folien schlauches (Figur 13 a) wird an seinem einen Ende mit einem einstückigen Flansch, der mit einer Siegelfolie verschlossen ist, verbunden (Figur 13 b). Der Behälter wird durch das offene andere Ende des Folienschlauches mit einem Fluid befüllt (Figur 13 c). Das offene Ende des Folienschlauches wird mit einer U-förmigen Quernaht verschlossen (Figur 13 d). Der gefüllte Behälter wird in eine Hülse aus Kunststoff geschoben (Figur 13 e). Der Rand der Kunststoffhülse wird auf den Rand des formstabilen Flansches aufgeschnappt. Das Fertigteil (Figur 13 f) ist zum Aufstecken auf einen Entnahmestutzen bereit.

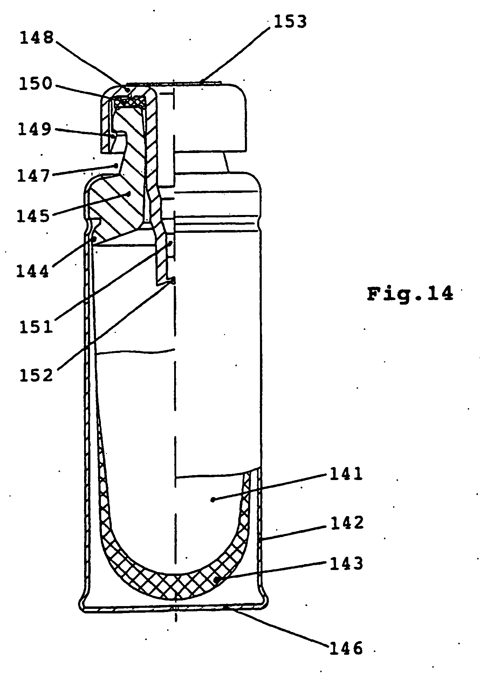

In Figur 14 ist ein typischer mit einem Fluid gefüllter erfindungs gemäßer Behälter (141) teilweise im Querschnitt dargestellt, der sich in einer Metallhülse (142) befindet. Der Folienbeutel ist an seinem einen Ende mit einer U-Naht (143) verschweißt und an seinem anderen Ende mit dem Rand (144) des Unterteils (145) eines zweistückigen zylindrischen formstabilen Flansches verschweißt. Die Metallhülse hat einen Boden (146), in dem sich ein Loch befindet, durch das Luft in den Raum zwischen Metallhülse und Folienbeutel eintreten kann. Das offene Ende der Hülse ist in eine Rille (147) im Rand des Unterteils (145) gedrückt. Die Hülse ist mit dem Flansch fest verbunden. Das Oberteil (148) des Flansches ist in eine Öffnung im Unterteil eingesetzt. Unterteil und Oberteil sind mit einem Schnappverschluß (149) verbunden und mit einer Flachdichtung (150) versehen. Der Führungskanal (151) ist an seinem inneren Ende mit einer Membran (152) verschlossen und an seinem äußeren Ende mit einer Siegelfolie (153) versiegelt.

Die in Figur 14 dargestellte Metallhülse besteht aus Aluminium. Sie ist 43 mm lang und hat einen Außendurchmesser von 17 mm und eine 0,5 mm dicke Wand. Der zweistückige Flansch besteht aus Polyethylen und ist im Spritzgußverfahren hergestellt. Das Oberteil des Flansches ist einschließlich Membran in einem Verfahrens schritt gefertigt. Der Führungskanal im Flanschoberteil hat an der Stelle der Preßpassung einen Innendurchmesser von 2,5 mm und paßt stramm auf einen Entnahmestutzen.

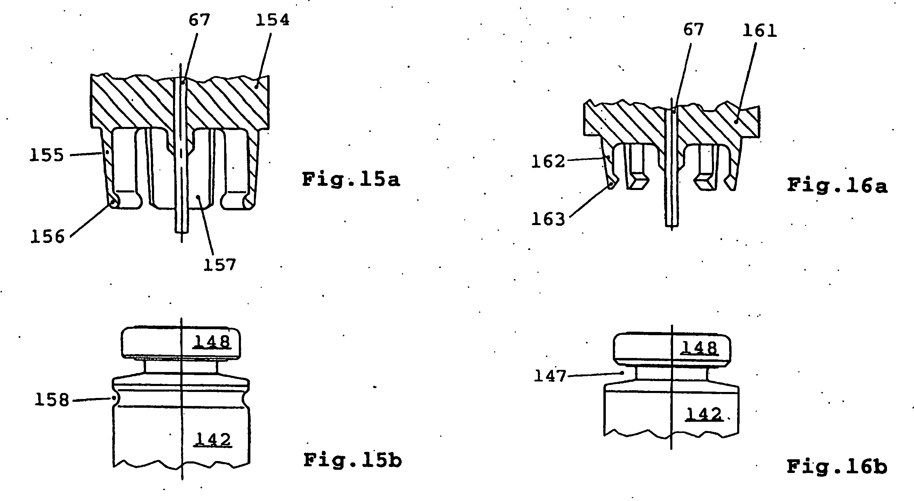

In den Figuren 15 a und 15 b ist eine lösbare formschlüssige steckbare Schnappverbindung zwischen dem formstabilen Flansch des Behälters und dem Anschlußstück in einem Entnahmegerät dargestellt.
Figur 15 a zeigt einen Querschnitt durch das im Entnahmegerät befindliche Anschlußstück (154), das auf seiner Achse den Entnahmestutzen (67) enthält. Der Entnahmestutzen ist umgeben von mehreren Schnapphaken (155) mit Schnappnasen (156) mit rundem Querschnitt. Die Schnapphaken sind durch Zwischenräume voneinander getrennt und können eine azimutale Breite von 10 Grad bis 60 Grad haben. Zwischen zwei Schnapphaken (155) kann ein Abschnitt (157) vorhanden sein, der keine Schnappnase enthält. Dieser Abschnitt legt sich formschlüssig an die Außenwand des eingesteckten Behälters an.
Figur 15 b zeigt in Seitenansicht das Ende eines in einer Hülse (142) befindlichen Behälters, dessen formstabiler Flansch (148) aus der Hülse herausragt. Die Hülse hat -entsprechend Figur 14 - bevorzugt im Bereich des in die Hülse hineinragenden Teils des Flansches eine umlaufende Rille (158), in die die Schnappnasen (156) des in das Anschlußstück (154) eingesteckten Behälters eingreifen, wodurch der Behälter mit dem Anschlußstück lösbar und formschlüssig verbunden wird.

In den Figuren 16 a und 16 b ist eine weitere Ausführungsform einer lösbaren, formschlüssigen, steckbaren Schnappverbindung dargestellt.
Figur 16 a zeigt einen Querschnitt durch das im Entnahmegerät befindliche Anschlußstück (161), das auf seiner Achse den Entnahmestutzen (67) enthält. Der Entnahmestutzen ist umgeben von mehreren zungenförmigen Schnapphaken (162) mit Schnappnasen (163), deren Flanken in beiden Bewegungsrichtungen des Behälters abgeschrägt sind. Die Schnappbaken sind in einem azimutalen Abstand vonein ander angebracht.
Figur 16 b zeigt in Seitenansicht das Ende eines in einer Hülse (142) befindlichen Behälters, dessen formstabiler Flansch (148) aus der Hülse herausragt. Die Schnappnasen (163) greifen bei in das Anschlußstück (154) eingestecktem Behälter in die umlaufende Rille (147) des aus der Hülse herausragenden Teils des form stabilen Flansches (148) ein, wodurch der Behälter mit dem Anschluß stück lösbar und formschlüssig verbunden wird.

## Patentansprüche

1. Verwendung eines gas- und flüssigkeitsdichten Behälters für eine medizinische Flüssigkeit in einem treibgasfreien Zerstäuber, welcher einen Entnahmestutzen (67) in Form eines Hohlkolbens aufweist, wobei der Behälter umfasst
- einen Folienbeutel (11,21,31), der an beiden Enden verschlossen ist, wobei mindestens ein Ende durch eine Schweißnaht (13, 23, 32) verschlossen ist, die im wesentlichen quer zur Beutelachse verläuft und der Folienbeutel bei einem Differenzdruck zwischen dem Innenraum des Behälters und seiner Umgebung unterhalb 300 hPa (300 mbar) durch den Außendruck verformbar ist,
- einen formstabilen Flansch (15, 25, 34), der an dem Folienbeutel dicht angebracht ist und der auf den Entnahmestutzen (67) aufgesteckt wird,
- einen Führungskanal (42, 54) in dem Flansch,
- wobei in dem Führungskanal eine Dichtstelle (56, 64, 74) ausgebildet ist und/oder eine Presspassung (55, 66, 77), die den Entnahmestutzen umschließt,
- und eine Entnahmestelle für die Flüssigkeit im Bereich des Führungskanals, in die bei Gebrauch der Hohlkolben sticht, so daß er in die medizinische Flüssigkeit eintaucht.

2. Verwendung nach Anspruch 1, wobei der Behälter **gekennzeichnet ist durch**
- einen Folienbeutel, der bei einem Differenzdruck unterhalb 150 hPa (150 mbar), bevorzugt unterhalb 80 hPa (80 mbar) verformbar ist.

3. Verwendung nach den Ansprüchen 1 und 2, wobei der Behälter **gekennzeichnet ist durch**
- einen Folienbeutel (31), der an beiden Enden **durch** eine Schweißnaht (32) verschlossen ist,
- und einen formstabilen Flansch (34), der am Folienbeutel (31) dicht angebracht ist.

4. Verwendung nach den Ansprüchen 1 und 2, wobei der Behälter **gekennzeichnet ist durch**
- einen Folienbeutel (11, 21), der an einem Ende **durch** eine Schweißnaht (13, 23) und am anderen Ende **durch** den formstabilen Flansch (12, 24) dicht verschlossen ist.

5. Verwendung nach den Ansprüchen 1 bis 4, wobei der Behälter **gekennzeichnet ist durch**
- einen formstabilen bevorzugt rotationssymmetrischen Flansch (41, 51, 61), dessen Größe an die Größe des Folienbeutels angepaßt ist,
- und mit dem der Folienbeutel an einem Ende verschlossen ist.

6. Verwendung nach den Ansprüchen 1 bis 5, wobei der Behälter **gekennzeichnet ist durch** einen Führungskanal (42, 54) mit einer Dichtstelle (56, 64, 74).

7. Verwendung nach den Ansprüchen 1 bis 6, wobei der Behälter **gekennzeichnet ist durch** einen formstabilen Flansch mit Preßpassung (55, 66, 77) innerhalb des Führungskanals, der den Entnahmestutzen umschliesst.

8. Verwendung nach den Ansprüchen 1 bis 7, wobei der Behälter **gekennzeichnet ist durch**
- einen formstabilen Flansch, der aus einem thermoplastischen Kunsstoff besteht.

9. Verwendung nach den Ansprüchen 1 bis 8, wobei der Behälter **gekennzeichnet ist durch**
- eine Schweißnaht (91, 101), die U- oder V-förmig ausgebildet ist und im wesentlichen quer zur Beutelachse verläuft, und die sich teilweise in Richtung der Beutelachse erstreckt, oder eine T-förmig ausgebildete Schweißnaht (111).

10. Verwendung nach den Ansprüchen 1 bis 9, wobei der Behälter **gekennzeichnet ist durch**
- eine Dichtstelle in Form eines in einer Nut liegenden Ringes (54, 74) aus elastischem Material.

11. Verwendung nach den Ansprüchen 1 und 10, wobei der Behälter **gekennzeichnet ist durch**
- eine Entnahmestelle, die als Einstichstelle ausgebildet ist.

12. Verwendung nach den Ansprüchen 1 bis 11, wobei der Behälter **gekennzeichnet ist durch**
- eine Entnahmestelle, die mit einer durchstechbaren Membran versehen ist.

13. Verwendung nach den Ansprüchen 1 bis 12, wobei der Behälter **gekennzeichnet ist durch**
- eine Entnahmestelle, die mit einer durchstechbaren Membran versehen ist, wobei
- die Membran (43, 78) am Ende oder innerhalb des Führungskanals angebracht ist,
- oder die Membran (86) ein Teil des Folienbeutels ist.

14. Verwendung nach den Ansprüchen 1 bis 13, wobei der Behälter **gekennzeichnet ist durch**
- eine Entnahmestelle, die mit einer Siegelfolie (58, 84, 151) versiegelt ist.

15. Verwendung nach den Ansprüchen 1 bis 14, wobei der Behälter **gekennzeichnet ist durch**
- einen Folienbeutel (11, 21, 31) aus einer Folie aus Kunststoff, Metall oder Metall-Legierung
- oder einen Folienbeutel aus einer Verbundfolie aus Kunststoff und Metall,
- oder einen Folienbeutel aus einer Kunststoff-Folie mit einer Schicht aus Metall, Glas oder Keramik.

16. Verwendung nach den Ansprüchen 1 bis 15, wobei der Behälter **gekennzeichnet ist durch**
- einen Folienbeutel mit
- einer Innenfolie aus Kunststoff und einer Außenfolie aus Metall
- oder mit zwei Folien aus unterschiedlichen Kunststoffen.

17. Verwendung nach den Ansprüchen 1 bis 15, wobei der Behälter **gekennzeichnet ist durch**
- einen Folienbeutel mit
- einer Innenfolie aus einem Copolymer, bevorzugt einem Polyethylen-Copolymer aus Ethylen-Acrylsäure,
- und einer diffusionsdichten Mittelfolie aus Metall oder Kunststoff oder einer diffusionsdichten Mittelschicht aus Metall, Glas oder Keramik,
- und einer Außenfolie aus Kunststoff, dessen Schmelztemperatur höher ist als die Schmelztemperatur der Innenfolie, bevorzugt aus Polyethylen-terephthalat.

18. Verwendung nach den Ansprüchen 1 bis 17, wobei der Behälter **gekennzeichnet ist durch**
- einen Folienbeutel aus einer Kunststoff-Folie mit einer Dicke von 20 µm bis 100 µm,
- oder einen Folienbeutel aus einer Verbundfolie mit einer Innenfolie aus Kunststoff mit einer Dicke von 20 µm bis 100 µm und einer Außenfolie aus Metall mit einer Dicke von 8 µm bis 20 µm,
- oder einen Folienbeutel aus einer Verbundfolie mit einer Innenfolie aus Kunststoff mit einer Dicke von 20 µm bis 100 µm, einer Mittelfolie aus Metall mit einer Dicke von 8 µm bis 20 µm und einer Außenfolie aus Kunststoff mit einer Dicke von 10 µm bis 40pm.

19. Verwendung nach den Ansprüchen 1 bis 18, wobei der Behälter **gekennzeichnet ist durch**
- ein Füllvolumen von 0,5 Milliliter bis 5 Milliliter, bevorzugt von 1 ml bis 4 ml.

20. Verwendung nach den Ansprüchen 1 bis 19, wobei der Behälter **gekennzeichnet ist durch**
- ein Füllvolumen von 1 ml bis 4 ml oder von 2 ml bis 4 ml.

21. Verwendung nach den Ansprüchen 1 bis 20, **dadurch gekennzeichnet, daß** sich der Behälter
- in einer formstabilen Hülse (142) aus Metall oder aus Kunststoff befindet, die mit dem Flansch verbunden ist.

22. Verwendung nach den Ansprüchen 1 bis 21, **dadurch gekennzeichnet, daß** sich der Behälter in einer formstabilen Hülse mit einem Durchmesser von 10 mm bis 30 mm, bevorzugt von 12 mm bis 17 mm befindet.

23. Verwendung nach den Ansprüchen 1 bis 22, **dadurch gekennzeichnet, daß** sich der Behälter in einer formstabilen Hülse befindet, aus der der formstabile Flansch hervorragt, wobei die Länge über alles von 20 mm bis 60 mm, bevorzugt von 30 mm bis 50 mm beträgt.

24. Verwendung nach den Ansprüchen 21 bis 23, wobei die Hülse **gekennzeichnet ist dadurch**, daß
- sie im wesentlichen rundum geschlossen ist,
- eine Öffnung enthält,
- oder bei der an der Verbindungsstelle mit dem Flansch ein Spalt vorhanden ist,
- oder daß sie als Korb mit vielen Öffnungen ausgebildet ist
- oder daß sie als Bügel ausgebildet ist.

25. Verwendung nach den Ansprüchen 21 bis 24, wobei der Behälter **dadurch gekennzeichnet ist, daß**
- er eine umlaufende Rille (158; 147) im formstabilen Flansch aufweist.

26. Verwendung des Behälters nach den Ansprüchen 1 bis 25
- als Primärverpackung für eine medizinische Flüssigkeit, die in vielen Teilmengen aus dem Folienbeutel entnommen wird.

27. Verwendung des Behälters nach den Ansprüchen 1 bis 26
- als Primärverpackung für eine medizinische Flüssigkeit, die jeweils in einer Dosis von 10 Mikroliter bis 50 Mikroliter, bevorzugt von 15 µl bis 20 µl portionsweise entnommen wird.

28. Verwendung des Behälters nach den Ansprüchen 1 bis 27
- als Kartusche, enthaltend eine inhalierbare Arzneimittel-Zubereitung, die als Lösung in Ethanol oder in Ethanol/Wasser oder in Wasser vorliegt.

29. Verwendung des Behälters nach den Ansprüchen 1 bis 28
- als Kartusche, enthaltend eine inhalierbare Arzneimittel-Zubereitung mit einem Wirkstoff oder mit mehreren Wirkstoffen, wie Berotec (Fenoterol-Hydrobromid;1-(3,5-dihydroxyphenyl)-2-[1-(4-hydroxy-benzyl)-ethyl]-amino]-ethanolhydrobromid), Atrovent (Ipratropiumbromid), Berodual (Kombination aus Fenoterol-Hydrobromid und Ipratropium-bromid), Salbutamol (oder Albuterol), Combivent, Oxivent (Oxitropiumbromid), Ba 679 (Tiotropiumbromid), BEA 2108 (Di- (2-thienyl)-glykolsäuretropenolester), Beclomethason, Flunisolid, Budesonid.

30. Verwendung des Behälters nach den Ansprüchen 1 bis 29
- mit einer Arzneimittel-Zubereitung zur Behandlung von Erkankungen mittels inhalierbarer Wirkstoffe.

31. Verwendung nach einem der Ansprüche 1 bis 30, **dadurch gekennzeichnet, daß**
- der Zerstäuber ein Entnahmegerät ist, das mit einem Anschlußstück versehen ist,
- in das der Behälter durch eine Schnappverbindung zwischen dem Anschlußstück (154; 161) im Entnahmegerät und dem formstabilen Flansch (148) des Behälters eingesteckt wird.

32. Verwendung nach Anspruch 31, **gekennzeichnet durch**
- eine umlaufende Rille (158; 147) im formstabilen Flansch, in die die am Anschlußstück (154; 161) angebrachten Schnappnasen (156; 163) der Schnapphaken (155; 162) bei in das Anschlußstück eingestecktem Behälter eingreifen.

33. Treibgasfreier Zerstäuber, welcher einen Entnahmestutzen in Form eines Hohlkolbens (67) zur Entnahme einer Flüssigkeit aufweist, der so mit einem Behälters für eine medizinische Flüssigkeit mit Merkmalen wie in einem der Ansprüche 1 bis 32 definiert, verbunden ist, dass der Hohlkolben durch die Entnahmestelle hindurch in den Behälter hineinragt.

## Claims

1. Use of a gas- and liquid-tight container for a medicinal liquid in a propellant-free atomiser, which comprises a dispensing connector (67) in the form of a hollow piston, said container comprising
- a foil bag (11, 21, 31) which is closed at both ends and wherein at least one end is sealed off by a weld seam (13, 23, 32), which extends substantially transversely with respect to the axis of the bag, and the foil bag is deformable by the external pressure at a differential pressure between the interior of the container and its environment of less than 300 hPa (300 mbar),
- a dimensionally stable flange (15, 25, 34) which is sealingly mounted on the foil bag and which is fitted onto the dispensing connector (67),
- a guide channel (42, 54) in the flange,
- wherein a sealing location (56, 64, 74) is formed in the guide channel and/or a press fit (55, 66, 77) which surrounds the dispensing connector,
- and a dispensing site for the liquid in the region of the guide channel into which the hollow piston penetrates during use so as to be immersed in the medicinal liquid.

2. Use according to claim 1, wherein the container is **characterised by**
- a foil bag which is deformable at a differential pressure below 150 hPa (150 mbar), preferably below 80 hPa (80 mbar).

3. Use according to claims 1 and 2, wherein the container is **characterised by**
- a foil bag (31) which is closed at both ends by a weld seam (32),
- and a dimensionally stable flange (34) which is sealingly mounted on the foil bag (31).

4. Use according to claims 1 and 2, wherein the container is **characterised by**
- a foil bag (11,21) which is sealed off at one end by a weld seam (13, 23) and at the other end by the dimensionally stable flange (12, 24).

5. Use according to claims 1 to 4, wherein the container is **characterised by**
- a preferably dimensionally stable, rotationally symmetrical flange (41, 51, 61), the size of which is adapted to the size of the foil bag,
- and with which the foil bag is closed at one end.

6. Use according to claims 1 to 5, wherein the container is **characterised by** a guide channel (42, 54) with a sealing location (56, 64, 74).

7. Use according to claims 1 to 6, wherein the container is **characterised by** a dimensionally stable flange with a press fit (55, 66, 77) within the guide channel which surrounds the dispensing connector.

8. Use according to claims 1 to 7, wherein the container is **characterised by**
- a dimensionally stable flange consisting of a thermoplastic plastics material.

9. Use according to claims 1 to 8, wherein the container is **characterised by**
- a weld seam (91, 101) which is of a U-shaped or V-shaped configuration and extends substantially transversely with respect to the axis of the bag and which extends partially in the direction of the axis of the bag, or a weld seam (111) of T-shaped configuration.

10. Use according to claims 1 to 9, wherein the container is **characterised by**
- a sealing location in the form of a ring (54, 74) of elastic material disposed in a groove.

11. Use according to claims 1 and 10, wherein the container is **characterised by**
- a dispensing site which is configured as a perforation point.

12. Use according to claims 1 to 11, wherein the container is **characterised by**
- a dispensing site which is provided with a pierceable membrane.

13. Use according to claims 1 to 12, wherein the container is **characterised by**
- a dispensing site which is provided with a pierceable membrane, wherein
- the membrane (43, 78) is disposed at the end of or inside the guide channel,
- or the membrane (86) is a part of the foil bag.

14. Use according to claims 1 to 13, wherein the container is **characterised by**
- a dispensing site which is sealed with a sealing foil (58, 84, 151).

15. Use according to claims 1 to 14, wherein the container is **characterised by**
- a foil bag (11, 21, 31) comprising a foil of plastics, metal or metal alloy
- or a foil bag comprising a composite foil of plastics and metal,
- or a foil bag comprising a plastics foil with a layer of metal, glass or ceramics.

16. Use according to claims 1 to 15, wherein the container is **characterised by**
- a foil bag with
- an inner foil of plastics and an outer foil of metal
- or with two foils of different plastics.

17. Use according to claims 1 to 15, wherein the container is **characterised by**
- a foil bag with
- an inner foil comprising a copolymer, preferably a polyethylene copolymer of ethylene-acrylic acid,
- and a diffusion-tight middle foil of metal or plastics or a diffusion-tight middle layer of metal, glass or ceramics,
- and an outer foil of plastics the melting temperature of which is higher than the melting temperature of the inner foil, preferably consisting of polyethylene terephthalate.

18. Use according to claims 1 to 17, wherein the container is **characterised by**
- a foil bag comprising a plastics foil with a thickness of 20 µm to 100 µm,
- or a foil bag comprising a composite foil with an inner foil of plastics having a thickness of 20 µm to 100 µm and an outer foil of metal with a thickness of 8 µm to 20 µm,
- or a foil bag comprising a composite foil having an inner foil of plastics with a thickness of 20 µm to 100 µm, a middle foil of metal having a thickness of 8 µm to 20 µm and an outer foil of plastics with a thickness of 10 µm to 40 µm.

19. Use according to claims 1 to 18, wherein the container is **characterised by**
- a fill capacity of 0.5 ml to 5 ml, preferably from 1 ml to 4 ml.

20. Use according to claims 1 to 19, wherein the container is **characterised by**
- a fill capacity of 1 ml to 4 ml or 2 ml to 4 ml.

21. Use according to claims 1 to 20, **characterised in that** the container
- is disposed in a dimensionally stable casing (142) made of metal or plastics, which is connected to the flange.

22. Use according to claims 1 to 21, **characterised in that** the container is disposed in a dimensionally stable casing having a diameter of 10 mm to 30 mm, preferably from 12 mm to 17 mm.

23. Use according to claims 1 to 22, **characterised in that** the container is disposed in a dimensionally stable casing from which the dimensionally stable flange projects, the overall length being from 20 mm to 60 mm, preferably from 30 mm to 50 mm.

24. Use according to claims 21 to 23, wherein the casing is **characterised in that**
- it is closed substantially all around,
- it contains an opening,
- or there is a gap at the point of connection to the flange,
- or it is constructed as a basket with a plurality of openings
- or it is constructed as a holder.

25. Use according to claims 21 to 24, wherein the container is **characterised in that**
- it comprises a peripherally extending groove (158, 147) in the dimensionally stable flange.

26. Use of the container according to claims 1 to 25
- as a primary packaging for a medicinal liquid which is removed from the foil bag in a plurality of partial amounts.

27. Use of the container according to claims 1 to 26
- as a primary packaging for a medicinal liquid which is removed in batches, in each case in a dosage of 10 microlitres to 50 microlitres, preferably from 15 µl to 20 µl.

28. Use of the container according to claims 1 to 27
- as a cartridge containing an inhalable medicament preparation which is present as a solution in ethanol or in ethanol/water or in water.

29. Use of the container according to claims 1 to 28
- as a cartridge containing an inhalable medicament preparation with one active substance or with a plurality of active substances, such as Berotec (fenoterol-hydrobromide;1-(3,5-dihydroxyphenyl)-2-[1-(4-hydroxy-benzyl)-ethyl]-amino]-ethanol hydrobromide), Atrovent (ipratropium bromide), Berodual (combination of fenoterol hydrobromide and ipratropium bromide), Salbutamol (or Albuterol), Combivent, Oxivent (oxitropium bromide), Ba 679 (tiotropium bromide), BEA 2108 (tropenol di-(2-thienyl)-glycolate), Beclomethasone, Flunisolide, Budesonide.

30. Use of the container according to claims 1 to 29
- with a medicament preparation for treating ailments by means of inhalable active substances.

31. Use according to one of claims 1 to 30, **characterised in that**
- the atomiser is a dispensing device which is provided with an attachment
- into which the container is inserted by a snap-fit connection between the attachment (154, 161) in the dispensing device and the dimensionally stable flange (148) of the container.

32. Use according to claim 31, **characterised by**
- a peripherally extending groove (158, 147) in the dimensionally stable flange into which the snap-in lugs (156, 163) of the snap-in hooks (155, 162) mounted on the attachment (154, 161) engage when the container is inserted into the attachment.

33. Propellant-free atomiser which comprises a dispensing connector in the form of a hollow piston (67) for dispensing a liquid which is connected to a container for a medicinal liquid having features as defined in one of claims 1 to 32 such that the hollow piston penetrates through the dispensing site into the container.

## Revendications

1. Utilisation d'un récipient étanche aux gaz et aux liquides pour un liquide médical dans un pulvérisateur sans gaz propulseur, qui présente un raccord de prise (67) en forme de piston creux, le récipient comprenant :
- un sachet en film (11, 21, 31), qui est fermé aux deux extrémités, au moins une extrémité étant fermée par une soudure (13, 23, 32), laquelle s'étend essentiellement de manière transversale par rapport à l'axe du sachet, et le sachet en film pouvant être déformé par la pression extérieure en cas de différence de pression entre l'intérieur du récipient et son environnement, inférieure à 300 hPa (300 mbar).
- une bride indéformable (15, 25, 34), qui est installée de manière étanche contre le sachet en film et qui est placée sur le raccord de prise (67),
- un canal de guidage (42, 54) dans la bride,
- un point d'étanchéité (56, 64, 74) étant configuré dans le canal de guidage et/ou un ajustage serré (55, 66, 77) qui entoure le raccord de prise,
- et un point de prise pour le liquide dans la zone du canal de guidage dans lequel le piston creux perce en cours d'utilisation, de telle manière qu'il s'immerge dans le liquide médical.

2. Utilisation selon la revendication 1, le récipient étant **caractérisé par**
un sachet en film, qui peut être déformé en cas de différence de pression inférieure à 150 hPa (150 mbar), de préférence inférieure à 80 hPa (80 mbar).

3. Utilisation selon les revendications 1 et 2, le récipient étant **caractérisé par**
- un sachet en film (31), qui est fermé au niveau des deux extrémités par une soudure (32),
- et une bride indéformable (34), qui est installée de manière étanche sur le sachet en film (31).

4. Utilisation selon les revendications 1 et 2, le récipient étant **caractérisé par**
- un sachet en film (11, 21), qui est fermé de manière étanche à une extrémité par une soudure (13, 23) et à l'autre extrémité par la bride non déformable (12, 24).

5. Utilisation selon les revendications 1 à 4, le récipient étant **caractérisé par**
- une bride indéformable, de préférence à symétrie de révolution (41, 51, 61), dont la taille est adaptée à la taille du sachet en film,
- et avec laquelle le sachet en film est fermé à une extrémité.

6. Utilisation selon les revendications 1 à 5, le récipient étant **caractérisé par** un canal de guidage (42, 54) avec un point d'étanchéité (56, 64, 74).

7. Utilisation selon les revendications 1 à 6, le récipient étant **caractérisé par** une bride indéformable avec ajustage serré (55, 66, 77) à l'intérieur du canal de guidage, qui entoure le raccord de prise.

8. Utilisation selon les revendications 1 à 7, le récipient étant **caractérisé par**
- une bride indéformable, qui se compose d'une matière synthétique thermoplastique.

9. Utilisation selon les revendications 1 à 8, le récipient étant **caractérisé par**
- une soudure (91, 101), qui est réalisée en forme de U ou de V et qui s'étend essentiellement de manière transversale par rapport à l'axe du sachet et qui s'étend partiellement dans le sens de l'axe du sachet, ou par une soudure configurée en forme de T (111).

10. Utilisation selon les revendications 1 à 9, le récipient étant **caractérisé par**
- un point d'étanchéité sous la forme d'une bague (54, 74) en matériau élastique, placée dans une rainure.

11. Utilisation selon les revendications 1 et 10, le récipient étant **caractérisé par**
- un point de prise qui est réalisé comme point de piqûre.

12. Utilisation selon les revendications 1 à 11, le récipient étant **caractérisé par**
- un point de prise qui est équipé d'une membrane perforable.

13. Utilisation selon les revendications 1 à 12, le récipient étant **caractérisé par**
- un point de prise qui est équipé d'une membrane perforable,
-- la membrane (43, 78) étant placée à l'extrémité ou à l'intérieur du canal de guidage,
-- ou la membrane (86) étant une partie du sachet en film.

14. Utilisation selon les revendications 1 à 13, le récipient étant **caractérisé par**
- un point de prise, qui est scellé par une feuille de scellement (58, 84, 151).

15. Utilisation selon les revendications 1 à 14, le récipient étant **caractérisé par**
- un sachet en film (11, 21, 31) fait d'une feuille de matière plastique, de métal ou d'alliage métallique
- ou un sachet en film fait d'une feuille multicouche en matière plastique et en métal,
- ou un sachet en film fait d'une feuille de matière plastique avec une couche de métal, de verre ou de céramique.

16. Utilisation selon les revendications 1 à 15, le récipient étant **caractérisé par**
- un sachet en film avec
-- une feuille intérieure en matière plastique et une feuille extérieure en métal
-- ou avec deux feuilles en matières plastiques différentes.

17. Utilisation selon les revendications 1 à 15, le récipient étant **caractérisé par**
- un sachet en film avec
- une feuille intérieure en copolymère, de préférence un copolymère polyéthylénique à base d'acide acrylique et d'éthylène,
- et une feuille centrale anti-diffusion en métal ou matière plastique ou une couche centrale anti-diffusion en métal, verre ou céramique,
- et une feuille extérieure en matière plastique, dont la température de fusion est supérieure à la température de fusion de la feuille intérieure, de préférence en poly(téréphtalate d'éthylène).

18. Utilisation selon les revendications 1 à 17, le récipient étant **caractérisé par**
- un sachet en film composé d'une feuille de matière plastique d'une épaisseur de 20 µm à 100 µm,
- ou d'un sachet en film composé d'une feuille multicouche avec une feuille intérieure en matière plastique d'une épaisseur de 20 µm à 100 µm et d'une feuille extérieure en métal d'une épaisseur de 8 µm à 20 µm,
- ou d'un sachet en film composé d'une feuille multicouche avec une feuille intérieure en matière plastique d'une épaisseur de 20 µm à 100 µm et d'une feuille centrale en métal d'une épaisseur de 8 µm à 20 µm et d'une feuille extérieure en matière plastique d'une épaisseur de 10 µm à 40 µm.

19. Utilisation selon les revendications 1 à 18, le récipient étant **caractérisé par**
- un volume de remplissage de 0,5 millilitre à 5 millilitres, de préférence de 1 ml à 4 ml.

20. Utilisation selon les revendications 1 à 19, le récipient étant **caractérisé par**
- un volume de remplissage de 1 ml à 4 ml ou de 2 ml à 4 ml.

21. Utilisation selon les revendications 1 à 20, **caractérisée en ce que** le récipient
- se trouve dans une gaine indéformable (142) en métal ou en matière plastique, qui est reliée à la bride.

22. Utilisation selon les revendications 1 à 21, **caractérisée en ce que** le récipient se trouve dans une gaine indéformable d'un diamètre de 10 mm à 30 mm, de préférence de 12 mm à 17 mm.

23. Utilisation selon les revendications 1 à 22, **caractérisée en ce que** le récipient se trouve dans une gaine indéformable, d'où sort la bride indéformable, la longueur hors tout étant de 20 mm à 60 mm, de préférence de 30 mm à 50 mm.

24. Utilisation selon les revendications 21 à 23, la gaine étant **caractérisé en ce**
- **qu'**elle est essentiellement complètement fermée,
-- **qu'**elle contient une ouverture,
-- ou dans laquelle une fente se trouve au point de raccordement avec la bride,
- ou qu'elle est configurée comme un panier avec de nombreuses ouvertures
- ou qu'elle est configurée comme un étrier

25. Utilisation selon les revendications 21 à 24, le récipient étant **caractérisé en ce qu'**il présente une nervure périphérique (158 ; 147) dans la bride indéformable.

26. Utilisation du récipient selon les revendications 1 à 25
- comme un emballage primaire pour un liquide médical, qui est prélevé en plusieurs quantités partielles à partir du sachet en film.

27. Utilisation du récipient selon les revendications 1 à 26
- comme un emballage primaire pour un liquide médical, qui est chaque fois prélevé par portions, en une dose de 10 microlitres à 50 microlitres, de préférence de 15 µl à 20 µl.

28. Utilisation du récipient selon les revendications 1 à 27
- comme une cartouche contenant une préparation médicamenteuse inhalable, sous forme d'une solution dans l'éthanol, dans de l'eau/éthanol ou dans l'eau.

29. Utilisation du récipient selon les revendications 1 à 28
- comme une cartouche contenant une préparation médicamenteuse inhalable avec un ou plusieurs principes actifs comme le Berotec (bromhydrate de fénotérol ; bromhydrate de 1-(3,5-dihydroxyphenyl)-2-[1-(4-hydroxy-benzyl)-éthyl]-amino] éthanol, Atrovent (Bromure d'ipratropium), Berodual (combinaison de bromhydrate de fénotérol et de bromure d'ipratropium), Salbutamol (ou Albutérol), Combivent, Oxivent (Oxitropiumbromure), Ba 679 (Titropiumbromure), BEA 2108 (Di-(2-thienyl) glycolate de tropénol), Beclomethason, Flunisolid, Budesonid.

30. Utilisation du récipient selon les revendications 1 à 29
- avec une préparation médicamenteuse pour le traitement de maladies par des principes actifs inhalables.

31. Utilisation selon l'une des revendications 1 à 30, **caractérisée en ce que**
- le pulvérisateur est un appareil de prise qui est équipé d'un embout de raccordement
- dans lequel le récipient est enfiché par un assemblage à encliquetage entre l'embout de raccordement (154, 161) dans l'appareil de prise et la bride indéformable (148) du récipient.

32. Utilisation selon la revendication 31, **caractérisée**
- une nervure périphérique (158 ; 147) dans la bride indéformable, dans laquelle s'engrènent les nez d'encliquetage (156, 163) installés sur l'embout de raccordement (154 ; 161) du crochet d'encliquetage (155 ; 162) dans le récipient embroché dans l'embout de raccordement.

33. Pulvérisateur sans gaz propulseur, qui présente un raccord de prise ayant la forme d'un piston creux (67) pour prélever un liquide, qui est relié à un récipient pour liquide médical avec des caractéristiques telles que définies dans les revendications 1 à 32, de telle manière que le piston creux avance à l'intérieur du récipient à travers le point de prise.
